# FASCICULE DE BREVET EUROPEEN

(11) **EP 0 841 909 B1**
(45) Date de publication et mention de la délivrance du brevet: **13.03.2002**
(21) Numéro de dépôt: 96927741.7
(22) Date de dépôt: 02.08.1996
(51) Int. Cl.: A61K 31/135, A61P 37/04

(54) **UTILISATION DE DIAMINES ALICYCLIQUES COMME IMMUNOMODULATEURS**
VERWENDUNG VON ALICYCLISCHEN DIAMINEN ALS IMMUNOMODULATOREN
USE OF ALICYCLIC DIAMINES AS IMMUNOMODULATORS

(30) Priorité: 03.08.1995 FR 9509459
(43) Date de publication de la demande: 20.05.1998
(73) Titulaire: NEOVACS, F-75003 Paris (FR)
(72) Inventeur: MAZIERE, Jean-Claude, F-91800 Brunoy (FR); ACHOUR, Amar, F-94000 Creteil (FR); LANDUREAU, Jean-Charles, F-77124 Villenoy (FR); ZAGURY, Daniel, F-75007 Paris (FR)
(74) Mandataire: Santarelli, Marc
(86) Numéro de dépôt international: FR9601242
(87) Numéro de publication internationale: WO9705859

(56) Documents cités:
- FR-A- 1 387 971
- SCIENCE, vol. 270, 15 Décembre 1995, pages 1811-1815, XP000616644 COCCHI, F. ET AL: "Identification of RANTES, MIP-1alpha, and MIP-1beta as the major HIV-suppressive factors produced by CD8+ T cells" cité dans la demande

## Description

La présente demande concerne de nouvelles utilisations de diamines alicycliques.

FR-A-1 387 971 décrit de nouvelles diamines alicycliques ainsi que leur procédé de préparation.

Les diamines alicycliques représentées par la formule de structure générale

R-NH-K-Q-K-NH-R (I)

dans laquelle Q représente un radical divalent répondant aux formules II à V suivantes: 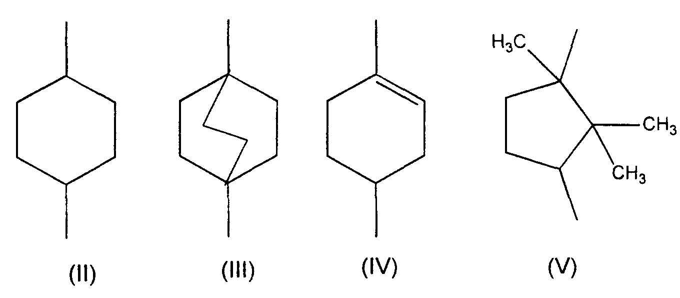 le symbole K représente un groupe méthylène ou, lorsque le symbole Q est représenté par la formule II, une valence de liaison et le symbole R est un radical choisi dans le groupe constitué par les radicaux VI à XVII suivants :

| | |
|---|---|
| VI | Benzyl |
| VII | Benzyl substitué par un halogène ou par un, deux ou trois groupes trihalométhyl |
| VIII | Benzyl trihalométhyl substitué. |
| IX | Indanyl |
| X | Alkyl |
| XI | Cycloalkyl |
| XII | Cycloalkylalkyl |
| XIII | Alcényl |
| XIV | Cycloalcénylalkyl |
| XV | Bicycloalkyl |
| XVI | Bicycloalcénylalkyl |
| XII | Bicycloalkylalkyl |

ainsi que leurs sels d'addition avec les acides minéraux ou organiques, présentent des propriétés affectant le métabolisme des lipides.

Après de longues recherches, la demanderesse a découvert que les produits définis ci-dessus présentaient de remarquables propriétés originales immunomodulatrices.

Les produits ci-dessus sont en effet notamment capables de restaurer l'expression de gènes de cytokines et/ou de leur récepteur en cas de dysfonctionnement de ces gènes lié à une pathologie d'origine exogène ou endogène. Ces propriétés sont illustrées ci-après dans la partie expérimentale.

Ils sont aussi capables de stimuler la production de molécules inflammatoires essentielles pour la défense de l'organisme humain comme les chimiokines (MIP 1 α, β, et Rantes dont les propriétés antivirales ont été décrites par exemple par Cocchi et al., Science, 270, 1811-1518, 1995).

Les diamines de formule I semblent agir comme un stimulateur des cytokines de type TH1 connues pour être perturbées en priorité dans le Sida (IL₂, IL₁₂ par exemple) ; elles pourraient par ce mécanisme limiter l'effet d'apoptose induit par le VIH et d'autres pathologies.

C'est pourquoi la présente invention a pour objet l'utilisation d'un composé répondant à la formule I telle que définie ci-dessus, à la préparation d'un médicament immunomodulateur, notamment capable de moduler l'expression des gènes des cytokines et/ou de leur récepteur en cas de dysfonctionnement lié à une pathologie d'origine exogène ou endogène, et à stimuler la production des cytokines.

Cette pathologie peut être liée à une agression virale telle que le SIDA de type I ou II, l'herpès, les hépatites, le papillome, ou à une agression parasitaire telle que la leishmaniose, la malaria ou la bilharziose. Elle peut être également d'origine iatrogène, par exemple induite par l'action d'immunodépresseurs tels que la cyclosporine ou par l'action des corticoïdes ainsi que par une tumeur, en particulier une tumeur hématologique comme le myélome, ou tissulaire.

La pathologie concernée est en particulier une maladie auto-immune telle que la polyarthrite rhumatoïde, le lupus érythémateux ou le diabète auto-immun.

Pour une définition plus détaillée des composés de formule I ci-dessus, ainsi que pour leur procédé de préparation et applications connues, on pourra se reporter utilement en particulier à FR-A-1 387 971 ainsi qu'à ses équivalents français ou étrangers, notamment au brevet spécial de médicament français N° 3559 M. Une abondante littérature concernant ces composés est également disponible, notamment pour le dichlorhydrate de trans 1-4 bis [2-chlorobenzyl aminométhyl]-cyclohexane encore connu sous le nom AY 9944.

En particulier, les données relatives à la pharmacologie et à la toxicologie de ce produit ont été largement décrites dans la littérature.

La présente invention a particulièrement pour objet l'utilisation d'un composé ci-dessus pour la préparation d'un médicament destiné au traitement
- d'une maladie virale,
- d'une maladie parasitaire,
- d'une maladie iatrogène,
- d'une tumeur.
- d'une maladie auto-immune

En vue de ces utilisations, on utilise en particulier un composé de formule I dans laquelle le symbole Q représente un radical 1,4 cyclohexanedi-yl, c'est à dire où le symbole Q correspond à la formule II précitée, notamment un composé de formule I dans laquelle le symbole R est un radical benzyl, ou un radical benzyl substitué par un halogène comme le fluor, le brome et notamment le chlore, ou par un, deux ou trois groupes trihalométhyl, ou encore un radical benzyl trihalométhyl substitué par un ou plusieurs, par exemple un, deux ou trois halogènes ou radicaux alkyle ou alkoxy en C₁-C₅, et tout particulièrement le composé AY 9944, à savoir le dichlorhydrate du composé de formule

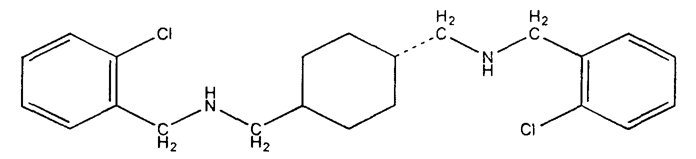

Ces propriétés de restauration de l'expression des gènes des cytokines et/ou de leur récepteur en cas de dysfonctionnement de ces gènes lié à une pathologie d'origine exogène ou endogène qui sont illustrées ci-après dans la partie expérimentale, justifient l'utilisation des dérivés de formule I ci-dessus décrits ainsi que de leurs sels, à titre de médicament destiné à corriger l'expression des gènes des cytokines et/ou de leur récepteur, en cas de dysfonctionnement lié à une pathologie d'origine exogène ou endogène.

Les médicaments selon la présente invention trouvent leur emploi par exemple dans le traitement, tant curatif que préventif, des pathologies évoquées ci-dessus.

Ils trouvent notamment leur emploi dans le traitement des maladies auto-immunes.

La dose usuelle, variable selon le sujet traité et l'affection en cause peut être par exemple de 10 mg à 2 g par jour par voie orale chez l'homme du composé connu sous le nom de AY 9944, aux besoins.

A titre de médicament, les composés répondant à la formule I ainsi que leurs sels ci-dessus peuvent être incorporés dans des compositions pharmaceutiques destinées à la voie digestive ou parentérale. Ces compositions pharmaceutiques peuvent être par exemple solides ou liquides et se présenter sous les formes pharmaceutiques couramment utilisées en médecine humaine, comme par exemple les comprimés simples ou dragéifiés, les gélules, les granulés, les caramels, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

On a en outre découvert que lorsque l'on associait un composé immunomodulateur de formule I tel que défini ci-dessus avec un composé à propriétés antivirales, tel que le DDI, le DDC, les antiprotéases, le 3 TC et de préférence l'AZT, on observait une potentialisation de leurs effets respectifs comme illustré ci-après dans la partie expérimentale.

C'est pourquoi la présente demande a aussi pour objet une association, caractérisée en ce qu'elle comprend un composé tel que défini ci-dessus, notamment le dichlorhydrate de trans 1-4 bis [2-chlorobenzyl aminométhyl]-cyclohexane et un composé à propriétés antivirales.

Le composé antiviral est de préférence choisi parmi le DDI, le DDC, les antiprotéases, le 3TC et notamment l'AZT.

La figure 1 montre l'effet de l'AY 9944 (AY), à la concentration finale de 3.10⁻⁶M, sur le nombre de cellules mononucléées présentes dans une culture de sang périphérique provenant d'un sujet sidéen (T4 = 400 / mm³). On trouve en abscisse le temps de culture en jours et en ordonnée le nombre de cellules par puits.

La figure 2 montre l'effet de l'AY 9944 à la concentration de 3.10⁻⁶M sur le pourcentage de cellules viables dans une culture de sang périphérique de sujet sidéen (T4 = 400 / mm³). On trouve en abscisse le temps de culture en jours et en ordonnée le pourcentage de mortalité cellulaire.

La figure 3 montre l'effet de l'AY 9944 à la concentration de 3.10⁻⁶M sur l'activité RT retrouvée dans le milieu en fonction du temps de culture.

La figure 4 montre l'effet de l'AY 9944 à la concentration de 3.10⁻⁶ M sur le pourcentage de cellules exprimant le récepteur de l'IL-2 dans des populations de lymphocytes de sang périphérique isolés à partir d'un sujet sain (témoin: Tém.) ou bien de différents patients sidéens (1 à 5).

La figure 5 montre l'effet d'un certain nombre de produits testés, à la concentration de 3.10⁻⁶ M, sur le pourcentage de lymphocytes de sang périphérique exprimant le CD4. Le témoin représente les lymphocytes de sang périphérique de sujets sidéens(T4 environ 400 / mm³) non traités.

La figure 6 représente l'effet des produits testés sur l'activité transcriptase reverse mesurée sur un extrait acellulaire obtenu à partir de cellules mononucléées d'un sujet sidéen.

La figure 7 représente l'effet des produits testés à la dose de 3.10⁻⁶M contre l'effet cytopathogène du virus HIV-1 III B vis à vis de lymphocytes obtenus à partir d'un sujet sain.

Les figures 8, 9, 10 et 11 représentent l'effet des produits testés à la dose de 3.10⁻⁶ M sur la synthèse des cytokines, respectivement L'IFN γ, Le MIP1α, le MIP1β, le Rantes.

Les exemples qui suivent illustrent la présente invention.

### PARTIE EXPERIMENTALE

Les cellules mononucléées provenant de sujets sidéens sont isolées sur gradient de Ficoll et activées par la phytohémagglutinine (PHA) durant 1 nuit, à 37°C. Par la suite, elles sont lavées, puis mises en culture à raison de 8x10⁵ / ml, en milieu RPMI supplémenté avec 10% de sérum de veau foetal décomplémenté, en présence de 40 Ul/ml d'interleukine-2 (recombinant, Roussel-Uclaf) et en présence ou non d'AY 9944 à la concentration testée, à savoir 3.10⁻⁶ M. La numération et le contrôle de la viabilité cellulaire (test au Bleu Trypan), sont effectués tous les 3 jours avec ou sans traitement par le ou les produits testés, et le milieu renouvelé.

Le typage des cellules est effectué périodiquement à l'aide d'un trieur de cellules ("FACS" Beckton-Dickinson), en utilisant des anticorps monoclonaux couplés à la fluorescéine dirigés soit contre l'antigène membranaire CD4 caractéristique des lymphocytes T auxiliaires.

### EXEMPLE 1

### Effet sur la prolifération cellulaire et sur le pourcentage de viabilité :

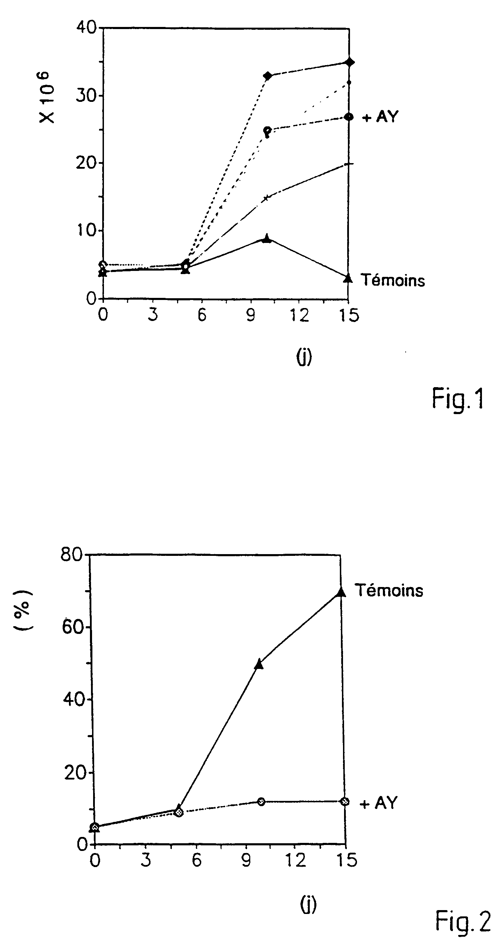

La figure 1 montre qu'alors que le nombre de cellules mononucléées présentes dans le sang provenant d'un donneur sidéen (T4: 400 / mm³) augmente peu, puis décroît après 10 jours (effet cytopathogène du virus et processus d'apoptose) dans les cellules infectées (témoins), on observe au contraire une prolifération spectaculaire dans les cultures infectées mais traitées par les molécules testées (AY: AY 9944) à la concentration de 3.10⁻⁶ M. C'est ainsi que, par exemple, pour l'AY 9944, après 15 jours de culture, le nombre de cellules est de 5 à 7 fois supérieur à celui des cultures non traitées.

Par ailleurs, on peut constater dans la Figure 2, ci-après, que par exemple dans le cas des cultures traitées par l'AY 9944, le pourcentage de cellules mortes dans la culture se maintient après infection aux alentours de 10%, alors qu'il atteint environ 70%, 15 jours après l'infection, dans le cas des cultures infectées mais non traitées (témoins). En outre, comme il est connu que ces cellules meurent par apoptose induite par le virus du Sida, on peut en conclure que l'AY 9944 a un effet anti-apoptogène. Cet effet anti-apoptogène est corroboré par les résultats des effets sur les cytokines ci-après.

Ainsi donc, la présence d'un composé de formule (I) dans le milieu de culture protège donc les cellules mononucléées de l'effet cytopathogène du virus, et permet une prolifération considérable en présence de lectine (PHA).

### EXEMPLE 2

### Effet sur l'activité transcriptase réverse ("Reverse Transcriptase": RT) retrouvée dans le milieu de culture:

L'activité de la transcriptase réverse (RT) est mesurée comme décrit par Barré-Sinoussi & al. (Science, 1983, 220:868). L'activité RT a été mesurée dans le milieu de culture des cellules infectées, à différents temps de culture.

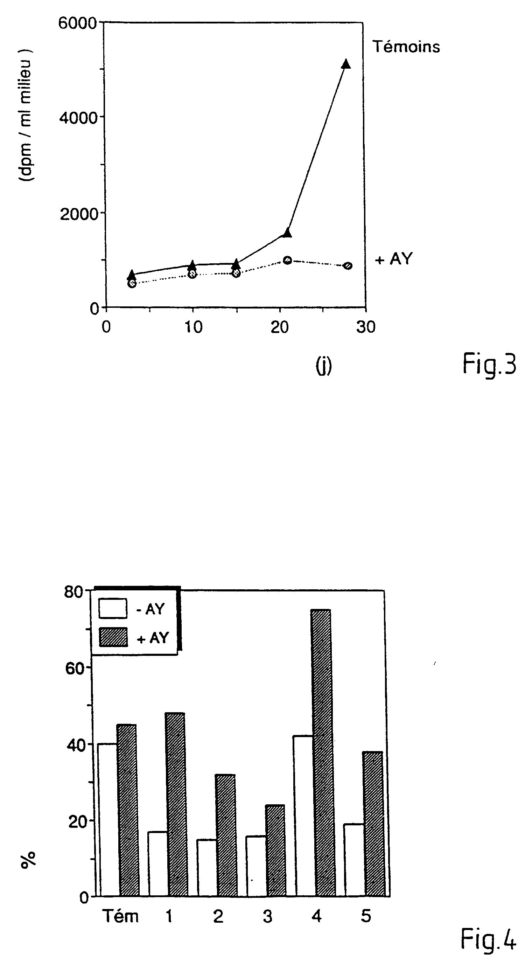

Les résultats présentés par la Figure 3, démontrent clairement que l'activité RT retrouvée dans le milieu demeure extrêmement basse dans le cas des cultures traitées par l'AY 9944, alors qu'elle augmente de plus de 1000 % dans les surnageants de cultures non traitées. Cet effet spectaculaire permet de penser que ce type de composés réduit drastiquement la charge virale. Par ailleurs, on verra ci-après qu'il ne s'agit pas d'une inhibition directe de l'enzyme (RT), mais bien d'une inhibition de la production virale, par d'autres mécanismes.

### EXEMPLE 3

### Effet sur l'expression des récepteurs de l'interleukine 2 (IL-2):

L'interleukine 2 est une cytokine jouant un rôle fondamental dans la régulation du système immunitaire, et plus particulièrement sur la multiplication des lymphocytes T auxiliaires, qui sont on le sait particulièrement affectés dans le SIDA. La Figure 4 met en évidence une stimulation particulièrement évidente de l'expression du récepteur de l'IL-2 au niveau des cultures traitées par l'AY 9944. Il est intéressant de noter que cette étude ayant été réalisée sur des lymphocytes de sang périphérique provenant de 5 patients différents, on observe dans tous les cas une forte augmentation du récepteur IL-2, y compris chez le patient n°4, chez lequel le degré d'expression du récepteur n'était pas significativement diminué. Dans ce dernier cas, on a donc une surexpression du récepteur de l'IL-2, par rapport au témoin (lymphocytes de sang périphérique d'un sujet normal). Pour les patients 1, 2 et 5, le pourcentage d'expression du récepteur passe de 15-18 (contre 40 chez le témoin) à 30-45%, c'est à dire que l'on a une véritable normalisation du degré d'expression de ce récepteur. On peut enfin noter que le composé testé n'a pas d'effet notable sur les lymphocytes de sang périphérique du sujet sain (témoin).

Cet effet est très important, l'IL-2 étant considérée comme l'une des cytokines jouant un rôle majeur dans l'immunité, la dysrégulation de sa production pouvant être l'un des points clés des troubles observés à la suite de l'infection par le HIV. La chute du taux des récepteurs de l'IL-2 est en effet bien corrélée à la numération des CD₄, ainsi qu'à la charge virale. Des expériences récentes montrent par ailleurs que la protéine virale d'expression précoce Tat, inhibe l'expression du messager de l'IL-2 (Chirmule & al. 1995, J.Virol. 69: 492).

### EXEMPLE 4

### Effet sur le pourcentage de cellules exprimant le CD4:

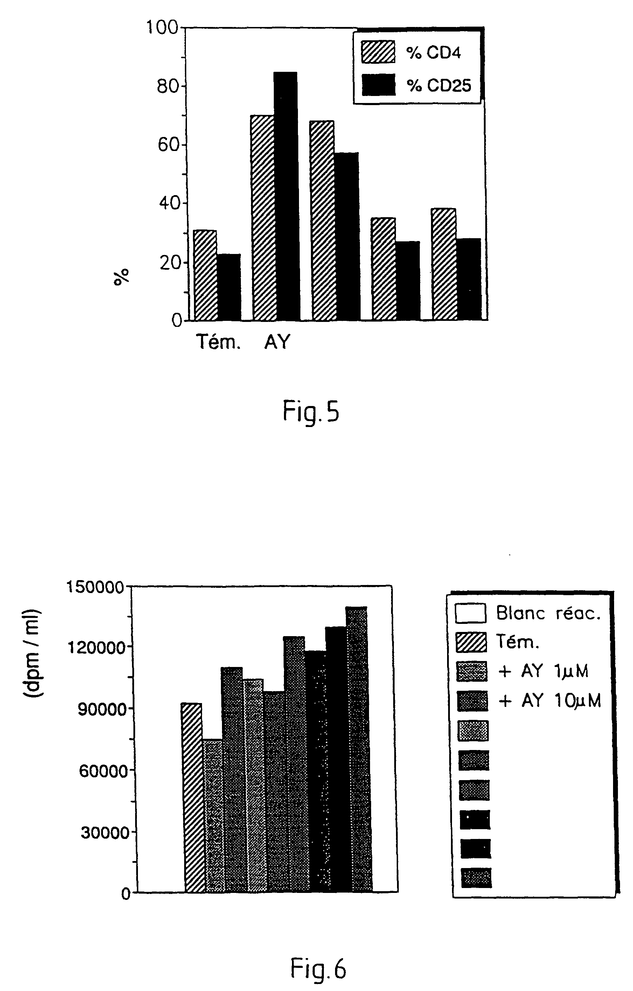

On sait que la diminution du nombre de lymphocytes T4, exprimant le marqueur CD4 est l'une des caractéristiques les plus évidentes de l'infection sidéenne, de telle sorte que la numération des "CD4" est devenue l'un des paramètres les plus utilisés pour le suivi de l'évolution de la maladie. La Figure 5 montre une remontée considérable du pourcentage des cellules exprimant le CD₄ (détecté par FACS à l'aide de l'anticorps fluorescent dirigé contre le CD₄). Dans cette série d'expériences, l'AY 9944 (AY) s'est avérée le plus efficace, faisant remonter le pourcentage de cellules CD₄⁺ d'environ 30% chez le témoin (lymphocytes de sang périphérique de sujet malade, non traités) à environ 70% pour les lymphocytes de sang périphérique cultivés en présence des deux produits précitées.

En résumé, on observe, après traitement par l'AY 9944, un effet positif sur la prolifération cellulaire et sur la viabilité cellulaire, une diminution de la charge virale, une stimulation de l'expression des messagers de l'interleukine 2 et du récepteur Tac de l'interleukine 2, enfin une augmentation du pourcentage de lymphocytes CD4⁺ avec rétablissement du rapport normal CD4/CD8. Il est à noter que des résultats similaires sont obtenus lorsque le produit testé est utilisé à la concentration de 10⁻⁷ M ou 10⁻⁸ M.

### EXEMPLE 5

### Effets au niveau transcriptionnel:

L'effet de l'AY 9944 a été étudié sur l'expression de l'ARN messager d'une interleukine jouant un rôle également important dans la prolifération des lymphocytes T, l'IL12 (sécrétée par les cellules présentatrices d'antigènes). 10⁶ cellules sont lysées, l'ADN extrait selon les techniques classiques, les ARN correspondants obtenus à l'aide de transcriptase réverse commerciale, puis amplifiés par PCR en utilisant les amorces correspondant aux ARN recherchés. Les résultats montrent que l'AY 9944 augmente l'expression de l'ARN messager codant pour l'IL12. La technique de PCR à été pratiquée avec deux amorces différentes de formules : 
TL 12 - P35 (+) 5' CAT GCT TTC AGA ATT CGG GC 3'
TL 12 - P35 (-) 5' GTT AGC TCA GAT GCT TTC ATG 3'
TL 12 - P 40 (+) 5' CCC TGA CAC CTG GAG TAC TC 3'
TL 12 - P 40 (-) 5' GGC TAT ACC ATG AAG CCT AG 3'
 correspondant aux deux sous-unités de l'IL12 (P40: pistes 1 à 4; P35: pistes 5 à 8). Les ARNm des la glycéraldéhyde 3 phosphate déshydrogénase (GAPDH) ont été étudiés en référence (pistes 9 à 12):

II apparaît donc que le produit testé agit entre autres mécanismes au niveau transcriptionnel, rétablissant en l'occurrence l'expression de certains gènes réprimés par les rétrovirus tels que le VIH-1. On observe donc une action en amont des perturbations précoces induites au niveau du génome par des agents pathogènes.

### EXEMPLE 6

### Effet au niveau de la différenciation cellulaire:

D'autres expériences ont été réalisées dans le but d'étudier l'effet de l'AY 9944 sur l'état de différenciation des cellules mononucléées. On a pu mettre en évidence une nette augmentation, après 6 jours de culture en présence d'AY 9944, du pourcentage des macrophages activés dans une population de cellules mononucléées obtenues à partir de sang périphérique d'un sujet sidéen.

### EXEMPLE 7

### Effet sur la transcriptase réverse mesurée in vitro (extrait acellulaire):

On a aussi vérifié que la diminution de l'activité RT mise en évidence dans les surnageants de cultures traitées par l'AY 9944 (voir Fig. 3) n'était pas due à un effet inhibiteur direct du produit. La figure 6 montre que, sur des extraits acellulaires, aucun des agents testés n'inhibe significativement l'activité RT, dans la zone de concentration utilisées (10⁻⁶ à 10⁻⁵M).

Les résultats obtenus avec l'AY 9944 sont représentés par les colonnes 3 et 4 à partir de la gauche, les autres colonnes vers la droite correspondent à d'autres produits testés.

En conclusion, les composés de formule (I) ne s'avèrent pas être des inhibiteurs de la transcriptase réverse. Ceci confirme le caractère original de leur activité, lesdits composés n'ayant pas de point d'action commun avec des médicaments actuels de type AZT

### EXEMPLE 8

### Protection in vitro des lymphocytes traités par les molécules amphiphiles cationiques, contre l'infection par le HIV:

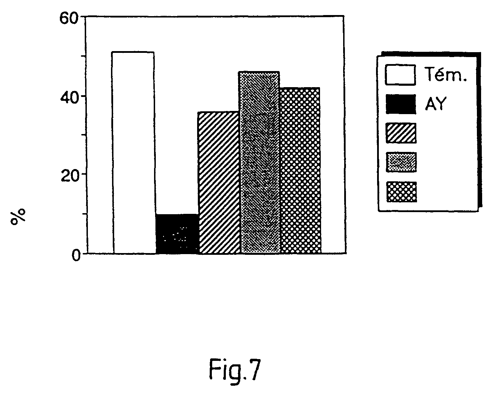

On a utilisé les lymphocytes provenant du sang périphérique d'un individu sain, infectés secondairement in vitro par une souche particulièrement virulente (HIV-IIIB) de façon à contrôler l'effet des produits testés contre une forte charge virale. La Figure 7 montre que, dans ce cas, les cellules ayant été prétraitées par les produits (3x10⁻⁶ M du produit testé) durant une semaine avant l'infection in vitro, l'AY 9944 protège les cellules de l'effet cytopathogène du virus.

### EXEMPLE 9 à 12

Les cellules mononucléées provenant de sujets sidéens sont isolées sur gradient de Ficoll et activées par la phytohémagglutinine (PHA) durant 1 nuit, à 37°C. Par la suite, elles sont lavées, puis mises en culture à raison de 8x10⁵ / ml, en milieu RPMI supplémenté avec 10% de sérum de veau foetal décomplémenté, en présence de 40 Ul/ml d'interleukine-2 (recombinant, Roussel-Uclaf) et en présence ou non d'AY 9944 à la concentration testée à savoir 3.10⁻⁶ M. La numération et le contrôle de la viabilité cellulaire (test au Bleu Trypan), sont effectués tous les 3 jours avec ou sans traitement par le ou les produits testés, et le milieu renouvelé.

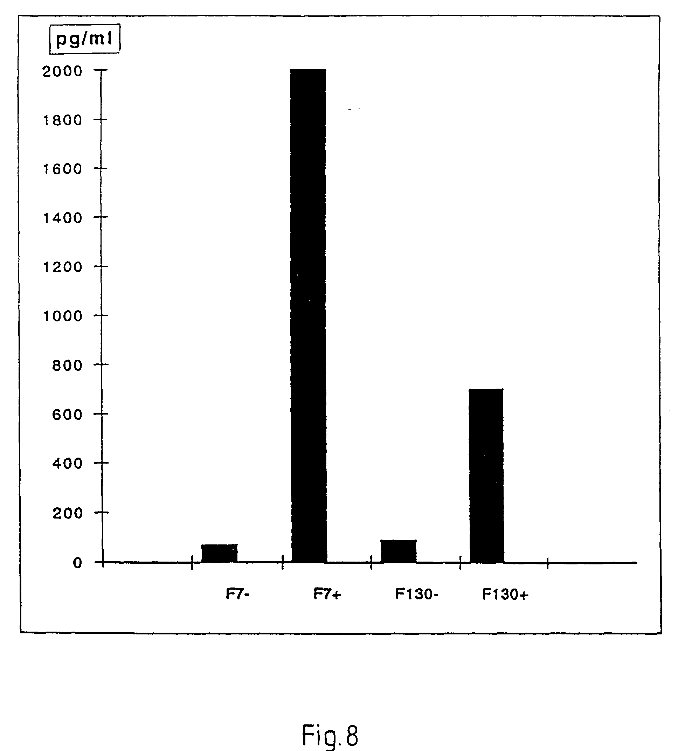

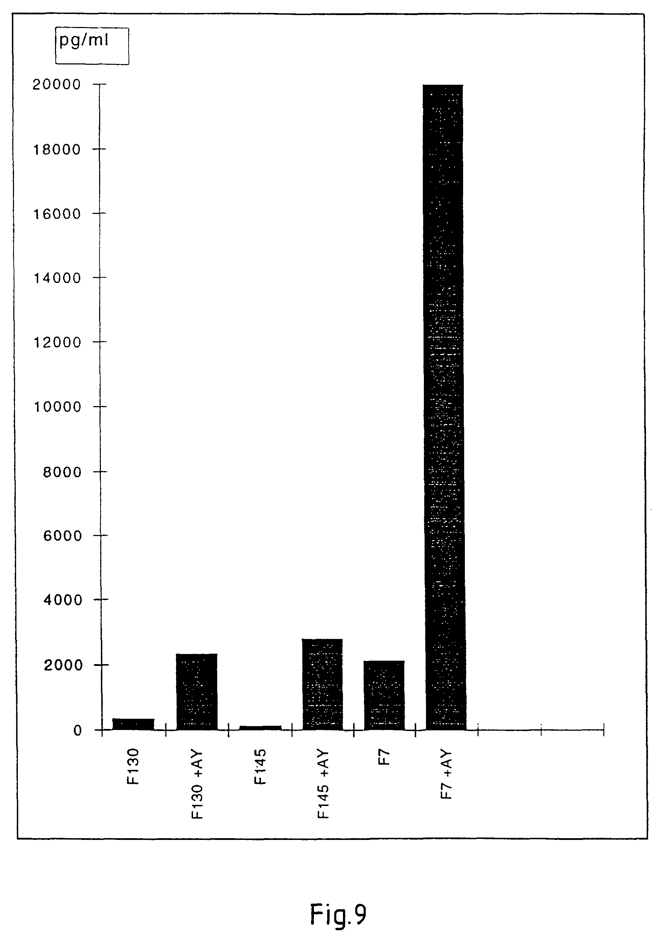

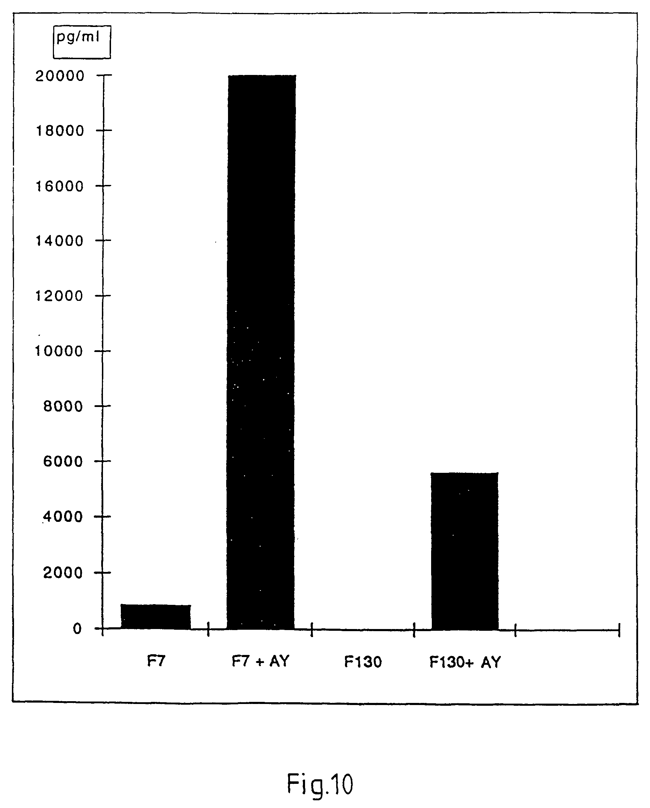

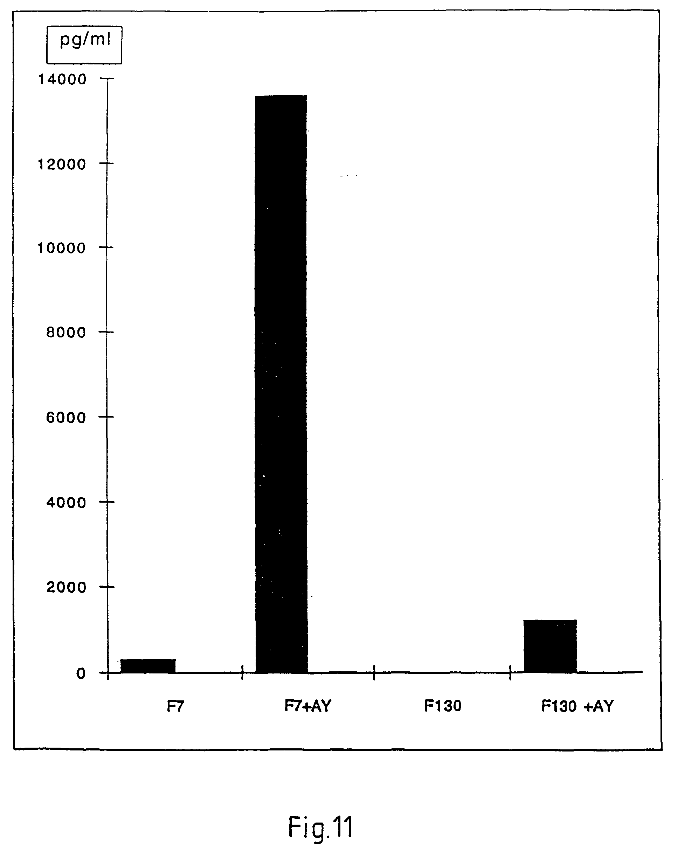

Le 10ème jour, on mesure la quantité respectivement d'IFN γ, de MIP1α, de MIP1β, de Rantes, produite dans le milieu de culture. Les résultats sont reportés sur les figures 8, 9, 10, 11. Les deux colonnes de gauche correspondent à un malade (F7) et les autres colonnes à un autre malade (F130). Pour chaque malade, la colonne de gauche (-) représente la production sans AY 9944, et celle de droite (+) représente la production avec l'AY 9944.

Dans tous les cas, on observe que la synthèse de ces cytokines est très fortement augmentée en présence d'AY 9944 et leur production est maintenue élevée durant les quatre semaines de culture.

### EXEMPLE 13

### Synergie entre l'AY 9944 et un composé antiviral

On infecte des cellules de lignée humaine H9 par la souche virale HIV-HTLV III B.

On reprend tous les trois jours les lymphocytes dans un milieu RPMI supplémenté de 10 % de sérum de veau foetal, à la concentration de 3.10⁵ cellules/ml.

On ajoute dans le milieu de l'AY 9944 , de l'AZT ou un mélange d'AY 9944 et d'AZT.

Après 30 jours de culture, on mesure la présence de virus par le test P 24, la prolifération cellulaire par incorporation de thymidine tritiée, la viabilité cellulaire par test d'exclusion au bleu Trypan, et le pourcentage de molécules CD4 exprimé par ces cellules.

Les résultats sont reportés dans le tableau ci-dessous - v signifie "sans virus", et +v signifie "avec virus".

On observe une synergie importante dès la dose de 3.10⁻⁸ M pour l'AY 9944 et 1 µg/ml pour l'AZT alors que même à la dose de 3.10⁻⁶ M, l'AY 9944 seul n'agit que faiblement (p24 = 70) et que l'AZT à la dose de 1µg/ml n'agit pas du tout (p24 > 1000).

On observe ainsi que l'AY 9944 a un effet restaurateur de la prolifération cellulaire inhibée par le virus.

On observe encore une baisse de la mortalité cellulaire (de 58% avec 1µg/ml d'AZT seul, à 11% en y ajoutant de l'AY 9944 à la concentration de seulement 3.10⁻⁸ M).

On observe enfin une restauration du phénotype CD4 qui passe de 26% à 83%, valeur normale, en ajoutant de l'AY 9944 à la concentration de 3.10⁻⁸ M à l'AZT (1µg/ml).

Les résultats sont présentés dans le tableau ci-dessous qui représente l'effet cumulé de l'AZT et de l'AY 9944 sur la multiplication du VIH, son effet lytique sur les lymphocytes et l'expression de l'antigène CD4. Ces résultats ont été obtenus après quatre semaines de culture de cellules IH9 infectées par l'isolat (HIV-1IIIB). Les lymphocytes sont passés tous les trois jours et ensemencés à la concentration de 3.10⁻⁵/ml.

Comme le montrent les résultats du tableau, l'AZT à 10 µg/ml, qui a un effet anti-viral (p24 = 0), a aussi un effet suppresseur (anti-prolifératif) sur les cellules de la lignée immune H9 car la prolifération cellulaire est diminuée à 28000 cpm pour l'AZT. Le fait d'ajouter de l'AY (ligne 6 du tableau) à cette concentration d'AZT permet de faire remonter la prolifération des cellules à 47000 cpm. Cela met en évidence l'effet restaurateur de l'AY 9944 sur la prolifération cellulaire face à l'activité d'une drogue qui a un effet anti-prolifératif (AZT 10µg/ml).

| | **P 24 (pg/ml)** | **Prolifération (cpm)** | **Viabilité (% cellules mortes)** | **% CD4** |
|---|---|---|---|---|
| - v | 0 | 63 000 | 9 | 82 |
| + v | 2 500 | 2 153 | 67 | 22 |
| + v (AZT 10 µg/ml) | 0 | 28 136 | 11 | 83 |
| + v (AZT 1 µg/ml) | 1 700 | 7 199 | 58 | 26 |
| + v (AY 9944 3.10⁻⁶ M) | 70 | 37 620 | 14 | 87 |
| + v (AY 9944 3.10⁻⁶ M; AZT 10 µg/ml) | 0 | 47 049 | 11 | 80 |
| + v (AY 9944 3.10⁻⁶ M; AZT 1 µg/ml) | 0 | 60 254 | 10 | 78 |
| + v (AY 9944 3.10⁻⁷ M; AZT 1 µg/ml) | 0 | 59 000 | 9 | 79 |
| + v (AY 9944 3.10⁻⁸ M; AZT 1 µg/ml) | 0 | 61 000 | 11 | 83 |

### EXEMPLE 14

On a préparé des comprimés répondant à la formule

| | |
|---|---|
| dichlorhydrate de trans 1-4 bis [2-chlorobenzyl aminométhyl]-cyclohexane | 10 mg |
| excipient q.s. pour un comprimé terminé à | 100 mg |
| (détail de l'excipient : lactose, amidon, talc, stéarate de magnésium) | |

### EXEMPLE 15 :

On a préparé des comprimés sécables répondant à la formule

| | |
|---|---|
| dichlorhydrate de trans 1-4 bis [2-chlorobenzyl aminométhyl]-cyclohexane | 15 mg |
| excipient q.s. pour un comprimé terminé à | 100 mg |
| (détail de l'excipient : lactose, amidon, talc, stéarate de magnésium). | |

### EXEMPLE 16 :

On a préparé des comprimés sécables répondant à la formule

| | |
|---|---|
| dichlorhydrate de trans 1-4 bis [2-chlorobenzyl aminométhyl]-cyclohexane | 15 mg |
| AZT | 100 mg |
| excipient q.s. pour un comprimé terminé à | 250 mg |
| (détail de l'excipient : lactose, amidon, talc, stéarate de magnésium). | |

## Revendications

1. Utilisation d'un composé répondant à la formule de structure générale
R - NH - K - Q - K - NH - R (I)
dans laquelle Q représente un radical divalent répondant aux formules II à V suivantes: 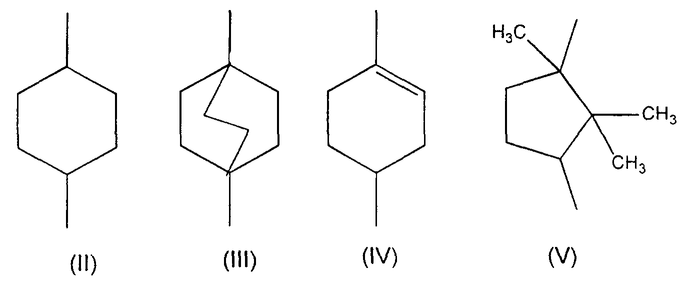 le symbole K représente un groupe méthylène ou, lorsque le symbole Q est représenté par la formule II, une valence de liaison. Le symbole R est un radical choisi dans le groupe constitué par les radicaux VI à XVII suivants :
| | |
|---|---|
| VI | Benzyl |
| VII | Benzyl substitué par un halogène ou par des groupes trihalométhyl |
| VIII | Benzyl trihalométhyl substitué. |
| IX | Indanyl |
| X | Alkyl |
| XI | Cycloalkyl |
| XII | Cycloalkylalkyl |
| XIII | Alcényl |
| XIV | Cycloalcénylalkyl |
| XV | Bicycloalkyl |
| XVI | Bicycloalcénylalkyl |
| XII | Bicycloalkylalkyl |
ainsi que leurs sels d'addition aux acides minéraux ou organiques, à la préparation d'un médicament immunomodulateur.

2. Utilisation selon la revendication 1, **caractérisée en ce que** l'immunomodulateur corrige l'expression des gènes des cytokines et/ou de leur récepteur en cas de dysfonctionnement lié à une pathologie d'origine exogène ou endogène.

3. Utilisation selon la revendication 2, **caractérisée en ce que** la pathologie est liée à une agression virale.

4. Utilisation selon la revendication 2, **caractérisée en ce que** la pathologie est liée à une agression parasitaire telle que la leishmaniose, la malaria ou la bilharziose.

5. Utilisation selon la revendication 2, **caractérisée en ce que** la pathologie est liée à une agression d'origine iatrogène, par exemple induite par l'action d'immunodépresseurs tels que la cyclosporine ou par l'action des corticoïdes.

6. Utilisation selon la revendication 2, **caractérisée en ce que** la pathologie est liée à une agression d'origine tumorale, telle qu'une tumeur hématologique comme le myélome, ou tissulaire.

7. Utilisation selon la revendication 2, **caractérisée en ce que** la pathologie est liée à une agression qui est une maladie auto-immune.

8. Utilisation selon la revendication 3, **caractérisée en ce que** la pathologie est liée à une agression d'origine virale due au HIV de type I ou II

9. Utilisation selon l'une des revendications 1 à 8, **caractérisée en ce que** le composé répond à la formule I dans laquelle Q représente un radical 1,4 cyclohexanedi-yl, K représente un groupe méthylène et R représente un radical benzyl ou un radical benzyl substitué par un halogène ou par un, deux ou trois groupes trihalométhyl ou un radical benzyl trihalométhyl substitué.

10. Utilisation selon l'une des revendications 1 à 8, **caractérisée en ce que** le composé de formule (I) est le dichlorhydrate de trans 1-4 bis [2-chlorobenzyl aminométhyl]-cyclohexane.

11. Une association d'un composé tel que défini à l'une des revendications 1, 9 et 10, et d'un composé à propriétés antivirales.

12. Une association d'un composé selon la revendication 11, **caractérisé en ce que** le composé antiviral est choisi parmi le DDI, le DDC, les antiprotéases, le 3TC et l'AZT.

## Claims

1. Use of a compound with the following general structural formula:
R - NH - K - Q - K - NH - R (I)
where Q represents a divalent radical with the following formulae II to V: 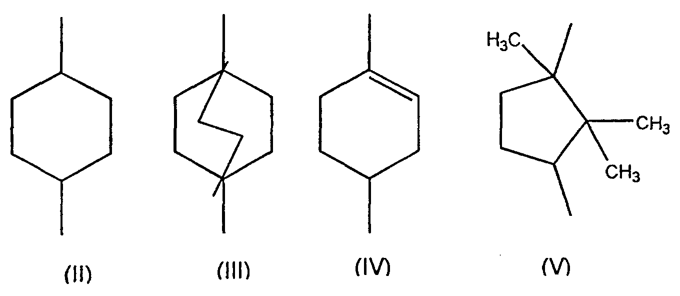 K represents a methylene group or, when the symbol Q is represented by formula II, a valence bond and the symbol R is a radical selected from the group formed by the following radicals VI to XVII:
| | |
|---|---|
| VI | Benzyl; |
| VII | Benzyl substituted by one, two or three trihalomethyl groups; |
| VIII | Substituted trihalomethyl benzyl; |
| IX | Indanyl; |
| X | Alkyl; |
| XI | Cycloalkyl; |
| XII | Cycloalkylalkyl; |
| XIII | Alkenyl; |
| XIV | Cycloalkenylalkyl; |
| XV | Bicycloalkyl; |
| XVI | Bicycloalkenylalkyl; |
| XII | Bicycloalkylalkyl; |
and their addition salts with mineral or organic acids, for the preparation of an immunomodulating drug.

2. Use according to claim 1 **characterized in that** the immunomodulator corrects the expression of cytokine genes and/or their receptor in the event of a dysfunction connected with an exogenous or endogenous pathology.

3. Use according to claim 2 **characterized in that** the pathology is connected with a viral infection.

4. Use according to claim 2 **characterized in that** the pathology is connected with a parasitic infection such as leishmaniosis, malaria or bilharziosis.

5. Use according to claim 2 **characterized in that** the pathology is connected with an infection of iatrogenic origin, for example induced by the action of immunodepressants such as cyclosporin or by the action of corticoids.

6. Use according to claim 2 **characterized in that** the pathology is connected with an infection of tumoral origin such as a haematological tumor such as a myeloma or a tissue tumor.

7. Use according to claim 2 **characterized in that** the pathology is connected with an infection which is an autoimmune disease.

8. Use according to claim 3 **characterized in that** the pathology is connected with an infection of viral origin due to HIV type I or II.

9. Use according to any one of claims 1 to 8 **characterized in that** the compound has formula I in which Q represents a 1,4-cycloheaxanedi-yl radical, K represents the methylene group and R represents a benzyl radical or a benzyl radical substituted with a halogen or by one, two or three trihalomethyl groups or a substituted trihalomethyl benzyl group.

10. Use according to any one of claims 1 to 8 **characterized in that** the compound with formula (I) is trans-1,4-bis[-chlorobenzylaminomethyl] cyclohexane dihydrochloride.

11. A combination of a compound as defined in any one of claims 1, 9 and 10 and a compound with antiviral properties.

12. A combination according to claim 11 **characterized in that** the antiviral compound is selected from DDI, DDC, antiproteases, 3TC and AZT.

## Patentansprüche

1. Verwendung einer Verbindung entsprechend der allgemeinen Strukturformel
R - NH - K - Q - K - NH - R (I)
worin Q einen zweiwertigen Rest entsprechend den nachfolgenden Formeln II bis V darstellt: 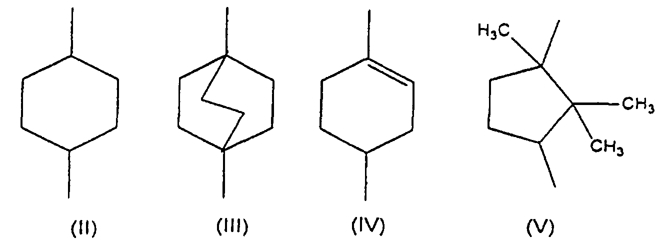 das Symbol K eine Methylengruppe darstellt oder, wenn das Symbol Q durch die Formel II dargestellt wird, eine Bindungsvalenz, das Symbol R einen Rest, ausgewählt aus der Gruppe, bestehend aus den nachfolgenden Resten VI bis XVII darstellt:
| | |
|---|---|
| VI | Benzyl |
| VII | mit einem Halogen oder durch Trihalogenmethylgruppen substituiertes Benzyl |
| VIII | Trihalogenmethyl-subsituiertes Benzyl |
| IX | Indanyl |
| X | Alkyl |
| XI | Cycloalkyl |
| XII | Cycloalkylalkyl |
| XIII | Alkenyl |
| XIV | Cycloalkenylalkyl |
| XV | Bicycloalkyl |
| XVI | Bicycloalkenylalkyl |
| XVII | Bicycloalkylalkyl |
genauso wie ihre Additionssalze von mineralischen oder organischen Säuren, zur Herstellung eines Immunmodulator-Medikaments.

2. Verwendung nach Anspruch 1, **dadurch gekennzeichnet, daß** der Immunmodulator die Expression von Cytokin-Genen und/oder ihres Rezeptors im Falle einer Funktionsstörung, die mit einer Pathologie von exogenem oder endogenem Ursprung verbunden ist, korrigiert.

3. Verwendung nach Anspruch 2, **dadurch gekennzeichnet, daß** die Pathologie mit einem viralen Angriff verbunden ist.

4. Verwendung nach Anspruch 2, **dadurch gekennzeichnet, daß** die Pathologie mit einem parasitären Angriff, wie der Leishmaniose, der Malaria oder der Bilharziose, verbunden ist.

5. Verwendung nach Anspruch 2, **dadurch gekennzeichnet, daß** die Pathologie mit einem Angriff iatrogenen Ursprungs, beispielsweise induziert durch die Wirkung von Immundepressoren, wie dem Cyclosporin, oder durch die Wirkung von Kortikoiden verbunden ist.

6. Verwendung nach Anspruch 2, **dadurch gekennzeichnet, daß** die Pathologie mit einem Angriff tumoralen Ursprungs, wie einem hämatologischen Tumor, wie dem Myelom, oder einem Gewebetumor verbunden ist.

7. Verwendung nach Anspruch 2, **dadurch gekennzeichnet, daß** die Pathologie mit einem Angriff verbunden ist, der eine Autoimmunerkrankung darstellt.

8. Verwendung nach Anspruch 3, **dadurch gekennzeichnet, daß** die Pathologie mit einen Angriff viralen Ursprungs aufgrund von HIV Typ I oder II verbunden ist.

9. Verwendung nach einem der Ansprüche 1 bis 8, **dadurch gekennzeichnet, daß** die Verbindung der Formel I entspricht, worin Q einen 1,4-Cyclohexandi-yl-Rest darstellt, K eine Methylengruppe darstellt und R einen Benzyl-Rest oder einen mit einem Halogen oder einen mit einer, zwei oder drei Trihalogenmethylgruppen substituierten Benzyl-Rest oder einen Trihalogenmethyl-substituierten Benzyl-Rest darstellt.

10. Verwendung nach einem der Ansprüche 1 bis 8, **dadurch gekennzeichnet, daß** die Verbindung der Formel (I) das Dichlorhydrat von trans-1,4-bis[2-Chlorbenzylaminomethyl]cyclohexan darstellt.

11. Assoziation einer Verbindung, wie nach einem der Ansprüche 1, 9 und 10 definiert, und einer Verbindung mit antiviralen Eigenschaften.

12. Assoziation einer Verbindung nach Anspruch 11, **dadurch gekennzeichnet, daß** die antivirale Verbindung ausgewählt ist aus DDI, DDC, den Antiproteasen, 3 TC und AZT.
